(19) **Europäisches Patentamt / European Patent Office / Office européen des brevets**

(11) **EP 3 412 149 A1**

(12) **EUROPEAN PATENT APPLICATION**
published in accordance with Art. 153(4) EPC

(43) Date of publication:
**12.12.2018 Bulletin 2018/50**

(21) Application number: **17747676.9**

(22) Date of filing: **25.01.2017**

(51) Int Cl.:
*A01N 1/02* *(2006.01)*
*C12N 5/0775* *(2010.01)*
*A61K 35/28* *(2015.01)*
*C12N 5/0783* *(2010.01)*
*A61K 35/17* *(2015.01)*

(86) International application number:
**PCT/KR2017/000859**

(87) International publication number:
**WO 2017/135631 (10.08.2017 Gazette 2017/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **01.02.2016 KR 20160012155**

(71) Applicant: **Green Cross Lab Cell Corporation Yongin-si, Gyeonggi-do 16924 (KR)**

(72) Inventors:
- **HWANG, Yu Kyeong**
  **Yongin-si**
  **Gyeonggi-do 16924 (KR)**

- **MIN, Bokyung**
  **Yongin-si**
  **Gyeonggi-do 16924 (KR)**
- **CHOI, Hana**
  **Yongin-si**
  **Gyeonggi-do 16924 (KR)**
- **KIM, Hyojin**
  **Yongin-si**
  **Gyeonggi-do 16924 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **MEDIUM COMPOSITION FOR CRYOPRESERVATION OF CELL AND USE THEREOF**

(57) The present invention relates to a medium composition for the cryopreservation of cells, which exhibits excellent cell recovery rates, cell viability rates and cell activity after thawing, and a pharmaceutical composition comprising the medium composition and therapeutic cells. Also, the medium composition of the present invention allows for readily administering the cryopreserved therapeutic cells to a subject without additional procedures such as washing, separation, etc. Therefore, the composition may be used as an excellent medium composition for cell cryopreservation or as an excellent therapeutic composition.

[Figure 1a]

**Description**

**Technical Field**

The present invention relates to a medium composition for cryopreservation of cells, which exhibits excellent cell recovery rates, cell viability rates and cell activity after thawing, and a pharmaceutical composition comprising the above medium composition and therapeutic cells.

**Background Art**

Along with the development of methods for culturing animal cells, such methods have been widely applied to biological studies of various fields including the production of protein drugs. When producing protein drugs using animal cells, cryopreservation is mainly used as a preservation method which allows maintaining of the characteristics of cell lines for a long time.

By cryopreservation, the difficulties in cell culture and maintenance can be overcome, and the contamination from bacteria, mycoplasma, and the like can be prevented. Further, the transformation of cell lines can be prevented, and also the reproducibility of experiments and production can be secured.

In general, animal cells are subject to cell damages during cryopreservation, that is, exposure of cells to a low temperature can result in impaired cellular organs and cellular functions. Therefore, in order to secure cell identity after cryopreservation, development and validation of a cryopreservation method that can maintain characteristics and viability rates of cells should be carried out.

As a general method used for cryopreservation of animal cells, 10% dimethyl sulfoxide (DMSO) is added to a cell culture medium supplemented with serum to protect cell membranes, and cells are gradually cooled and stored in liquid nitrogen at -196°C. However, the frequency of the use of such method in a process such as protein production is decreasing due to the problems of serum. In addition, although trace elements in serum impose influences on the growth of cells, there are problems that it is difficult to analyze such elements, and the elements may vary depending on the production time and place.

In addition, most of the serum is derived from an animal, and even if it is derived from a human, it is exposed to infection by viruses or the like. Therefore, there is a safety problem in using a composition containing the same as a direct therapeutic drug such as a cell therapeutic agent.

**Disclosure of Invention**

**Technical Problem**

Accordingly, the present inventors established a medium composition for cryopreservation that allows long-term cryopreservation of animal cells and is free of serum and thus useful as a composition for cell therapeutic agent, and have completed the present invention.

Accordingly, an object of the present invention is to provide a medium composition for cryopreservation of cells.

Another object of the present invention is to provide a pharmaceutical composition comprising the above medium composition for cryopreservation.

**Solution to Problem**

In accordance with one object of the present invention, there is provided a medium composition for cryopreservation of cells comprising 15 to 55 v/v% of albumin, 20 to 30 v/v% of dextran, 2 to 8 v/v% of dimethyl sulfoxide and 15 to 55 v/v% of a cell culture medium based on the total volume of the composition.

In accordance with another object of the present invention, there is provided a pharmaceutical composition for preventing or treating a cancer or infectious disorder comprising the medium composition of the present invention and immune cells as active ingredients.

In accordance with still another object of the present invention, there is provided a pharmaceutical composition for preventing or treating a degenerative disorder and immune-related disorder comprising the medium composition of the present invention and stem cells as active ingredients.

**Advantageous Effects of Invention**

If cells are frozen in a medium composition for cryopreservation of cells according to the present invention, the viability rates of the cells are high when the cells are thawed, and also the activities of the cells are maintained. In addition, the medium composition of the present invention allows administering of the cryopreserved therapeutic cells to a subject without additional procedures such as washing, separation, etc. Therefore, the composition may be used as an excellent medium composition for cryopreservation of cells or as an excellent therapeutic composition.

**Brief Description of Drawings**

Figs. 1a to 1d show the recovery rates (Figs. 1a and 1c) and viability rates (Fig. 1b and Fig. 1d) after thawing NK cells which were frozen in the medium composition for cryopreservation of cells prepared in an embodiment of the present invention and culturing with IL-2.

Figs. 2a to 2d show the cytotoxicity on K562 cells of NK cells frozen in the medium composition for cryopreservation of cells prepared according to an embodiment of the present invention, in which Figs. 2a and 2b show the measurement values immediately after thawing the cells and Fig. 2c and Fig. 2d show the measurement values after thawing and culturing with IL-2.

Figs. 3a and 3b show the cell numbers (Fig. 3a) and viability rates (Fig. 3b) measured after thawing HuT78 cells frozen in a medium composition for cryopreservation of cells prepared in an embodiment of the present invention and culturing for 3 days.

Figs. 4a and 4b show the cell numbers (Fig. 4a) and viability rates (Fig. 4b) measured after thawing K562 cells frozen in a medium composition for cryopreservation of cells prepared in an embodiment of the present invention and culturing for 3 days.

**Best Mode for Carrying out the Invention**

Hereinafter, the present invention will be described in detail.

The present invention provides a medium composition for cryopreservation of cells comprising 15 to 55 v/v% of albumin, 20 to 30 v/v% of dextran, 2 to 8 v/v% of dimethyl sulfoxide and 15 to 55 v/v% of a cell culture medium based on the total composition.

As used herein, the term "cryopreservation" refers to stably maintaining cells for a long period of time via freezing. Generally, when cells are cultured, a mutation occurs in a ratio of one to ten thousand, and when cells go through a long-term subculture, cell populations may change and become different from the original populations. In severe cases, cells may lose their own particular functions by subculture. Further, cells may be infected with mycoplasma or the like during subculture. Because of such problems, cell cryopreservation is performed to freeze and preserve cells before losing their intrinsic characteristics, and to use them when needed.

Cell cryopreservation may be performed by treating a cryportectant to cells to be cryopreserved, and a composition of the present invention includes albumin, dextran, dimethyl sulfoxide and a cell culture medium to prevent cell damage due to a cryoprotectant, thereby improving safety and stability in cryopreservation of cells.

The term "cryoprotectant" as used herein refers to a substance used when the cells are stored at an ultra-low temperature of -80°C to -200°C. In particular, the substance can minimize the formation of ice crystals and cell damage due to imbalance of ion and osmotic pressure inevitably accompanied by freezing and thawing processes.

As used herein, the term "albumin" refers to a total human albumin or a portion of human albumin that has biologically activity. The albumin can be used as an animal-free substitute which is equivalent to or superior to serum in cryopreservation and is added to the composition for cryopreservation to stabilize cells.

The albumin may be a protein having an Accession Number of ANC98520.1 in GenBank of the US National Institutes of Health, and may include any amino acid sequences exhibiting similarity to the above protein of 70% or more, specifically 80% or more, more specifically 95% or more, most specifically 98% or more. In addition, a protein variant having an amino acid sequence in which a partial sequence is deleted, modified, substituted, or added may also included, as long as the amino acid sequence has a biological activity equivalent or corresponding to that of the above albumin

The content of albumin contained in the composition of the present invention may be 15 to 55 v/v%, specifically 18 to 40 v/v%, more specifically 20 to 35 v/v% based on the total volume of the composition. According to one embodiment of the present invention, the content of albumin may be 20 v/v%, 35 v/v% or 50 v/v% based on the total composition. Herein, the "v/v%" refers to the volume percentage of each component based on the volume of the total composition.

As used herein, the term "dextran" refers to a polysaccharide which is a polymer of D-glucose. The dextran can be classified into dextran 1, dextran 40, dextran 70, and the like, according to the weight average molecular weight. In one embodiment, the dextran may be dextran 40.

The content of dextran contained in a composition of the present invention may be 20 to 30 v/v%, specifically 23 to 27 v/v%, based on the total volume of the composition. According to one embodiment of the present invention, the dextran content may be 25 v/v% based on the total composition.

As used herein, the "dimethyl sulfoxide (DMSO)" is used as a cell membrane permeable cryopreservation agent. It permeates the cell membrane to replace intracellular water and inhibits the formation of ice crystals during freezing.

The content of dimethyl sulfoxide contained in the composition of the present invention may be 2 to 8 v/v%, specifically 4 to 6 v/v%, based on the total volume of the composition. According to one embodiment of the present invention, the content of dimethyl sulfoxide may be 5 v/v% based on the total composition.

As used herein, the term "cell culture medium" refers to a mixture for growth and proliferation of cells *in vitro*, which contains essential elements for growth and proliferation of cells such as sugars, amino acids, various nutrients, inorganic substances, etc. The cell culture medium may be optimized for specific cell culture, for example, a basic culture medium prepared for sustaining cell growth, a cell culture production medium prepared to promote protein production from cells, a concentration medium prepared by concentrating nutrients in a high concentration.

The above cell culture medium may contain at least one ingredient selected from the group consisting of L-alanine, L-arginine hydrochloride, L-cysteine hydrochloride-monohydrate, L-glutamine, L-histidine hydrochloride-monohydrate, L-lysine hydrochloride, L-methionine, L-proline, L-serine, L-threonine, L-valine, L-asparagine-monohydrate, L-aspartic acid, L-cysteine 2HCl, L-glutamic acid, L-isoleucine, L-leucine, L-phenylalanine, L-tryptophan, L-tyrosine disodium dihydrate, i-inositol, thiamine hydrochloride, niacinamide, pyridoxine hydrochloride, biotin, calcium D-pantothenate, folic acid, riboflavin, vitamin B12, sodium chloride (NaCl), sodium bicarbonate (NaHCO$_3$), potassium chloride (KCl), calcium chloride (CaCl$_2$), sodium hydrogen phosphate monohydrate (NaH$_2$PO$_4$-H$_2$O), copper sulfate pentahydrate (CuSO$_4$-5H$_2$O), ferric sulfate heptahydrate (FeSO$_4$-7H$_2$O), magnesium chloride (anhydrous), magnesium sulfate (MgSO$_4$), disodium hydrogen phosphate (Na$_2$HPO$_4$), zinc sulfate heptahydrate (ZnSO$_4$-7H$_2$O), D-glucose (dextrose), sodium pyruvate, hypoxanthin Na, linolenic acid, lipoic acid, putrescine 2HCl and thymidine.

The cell culture medium according to the present invention may be prepared by artificial production or commercially available medium may be used. Examples of the commercially available cell culture media include DMEM (Dulbecco's Modified Eagle's Medium), MEM (Minimal Essential Medium), BME (Basal Medium Eagle), RPMI1640, F-10, F-12, α-MEM (α-Minimal Essential Medium), G-MEM (Glasgow's Minimal Essential Medium), cellgro SCGM, X-VIVO, AIM-V, etc.

The content of the culture medium may be 15 to 55 v/v%, specifically 30 to 52 v/v%, based on the total volume of the composition. According to one embodiment of the present invention, the content of the culture medium may be 20 v/v%, 35 v/v%, or 50 v/v% based on 100 v/v% of the total composition.

The cells that can be preserved with a composition for cryopreservation of the present invention may be animal cells, but any cells that can be stably cryopreserved by the composition of the present invention can be used. The cells may be derived from a human, a monkey, a pig, a horse, a cow, a sheep, a dog, a cat, a mouse, a rabbit or the like, and specifically, may be immune cells, tumor cells, cord blood cells, stem cells, epithelial cells, primary cells, and the like which are derived from the animal.

In addition, a composition for cryopreservation of the present invention may further comprise a buffer, an isotonic agent or an apoptosis inhibitor. The buffer may be any one selected from the group consisting of citrate, phosphate, succinate, tartrate, fumarate, gluconate, oxalate, lactate, acetate, histidine and tris. Meanwhile, the isotonic agent may be selected from the group consisting of citrate, phosphate, succinate, tartrate, fumarate, gluconate, oxalate, lactate, acetate, histidine, and tris. Meahwhile the isotonic agent may be one selected from the group consisting of sodium chloride, potassium chloride, boric acid, sodium borate, mannitol, glycerin, propylene glycol, polyethylene glycol, maltose, sucrose, erythritol, arabitol, xylitol, sorbitol trehalose, and glucose. The apoptosis inhibitor may be one selected from the group consisting of a Rho associated kinase (ROCK) inhibitor, catalase, and zVAD-fmk.

The present invention provides a method for cryopreservation of cells, comprising the step of freezing cells to which a composition for cryopreservation is added.

The freezing of cells to which a composition for cryopreservation according to the present invention is added can be conducted by a conventional method, e.g., vitrification freezing, slow freezing, and the like. Vitrification freezing is a method of freezing cells by controlling the temperature at a constant rate per minute using a device such as controlled rate freezing (CRF). When the temperature reaches -80°C, the frozen cells are stored in a nitrogen tank. Meanwhile, slow freezing is a method of freezing cells by placing a vial containing a composition for cryopreservation containing cells into a container box for freezing containing isopropyl alcohol and then dropping the temperature at a constant rate for 12 to 24 hours in an ultra-low temperature freezer at -70°C or lower. Thereafter, the frozen cells are stored in a liquid nitrogen tank.

According to one embodiment of the present invention, the method for cryopreservation of cells may be vitrification freezing. The vitrification freezing is performed using CRF by 1) cooling the cells at room temperature to 4°C; 2) cooling to - 8°C at -1°C per minute; 3) cooling to -65°C at -25°C per minute; 4) raising the temperature to -14°C at 15°C per minute; 5) cooling to -45°C at -1°C per minute; and 6) cooling to -90°C at -10°C per minute.

The above method for cryopreservation of cells can be carried out by containing cells in an appropriate concentration in a vial. The concentration of the cells may be 1 x 10$^4$ to 1 x 10$^8$ cells/ml, specifically, 1 x 10$^5$ to 1 x 10$^7$

cells/ml per vial, but the concentration may vary depending on the type of cells.

The present invention provides a pharmaceutical composition for preventing or treating a cancer or infectious disorder comprising a composition of the present invention and immune cells as active ingredients.

The composition is the same as the above-described medium composition for cryopreservation of cells. However, when used as a pharmaceutical composition, the cell culture medium which can be comprised is preferably a medium supplemented with no toxic substance such as serum, phenol red or β-mercaptoethanol. Accordingly, the pharmaceutical composition of the present invention has no human toxicity, and thus can be directly administered to a subject after thawing.

As used herein, the term "immune cell" refers to the cell that participates in the immune response of the human body, including natural killer cells, T cells, B cells, and dendritic cells, etc. Specifically, the immune cells may be natural killer cells.

The above cancer may include bladder cancer, breast cancer, stomach cancer, lung cancer, ovarian cancer, thyroid cancer, uterine cervix cancer, central nervous system cancer, glioma, liver cancer, skin cancer, pancreatic cancer, gastric cancer, colon cancer, rectal cancer, esophageal cancer, renal cancer, lung cancer, epithelial cancer, blood cancer, prostate cancer, soft tissue sarcoma, multiple sclerosis, and the like.

As used herein, the term "infectious disorder" refers to a pathological state caused by infection of viruses or pathogens, including all disorders that may be transmitted or infected through respiratory tract, blood, skin contact, etc. Examples of such infectious disorders include, but are not limited to, hepatitis B and C, human papilloma virus (HPV) infection, cytomegalovirus infection, a viral respiratory disorder, influenza, and the like.

The present invention also provides a pharmaceutical composition for preventing or treating a degenerative disorder comprising the composition of the present invention and stem cells as active ingredients.

The composition is the same as the above-described medium composition for cryopreservation of cells. However, when used as a pharmaceutical composition, the cell culture medium which can be comprised is preferably a medium supplemented with no toxic substance such as serum, phenol red or β-mercaptoethanol. Accordingly, the pharmaceutical composition of the present invention has no human toxicity, and thus can be directly administered to a subject after thawing.

As used herein, the term "stem cell" refers to cells having pluripotency capable of differentiating into various cells. Such stem cells may include human embryonic stem cells, bone marrow stem cells, mesenchymal stem cells, human neural stem cells, oral mucosal lamina propria stem cells, and the like. Specifically, the stem cells may be mesenchymal stem cells.

As used herein, the term "degenerative disorder" refers to a pathological state in which the tissue has lost its original function due to irreversible quantitative loss of the tissue. Such degenerative disorder includes, but are not limited to, a cranial nerve disorder, an ischemic disorder, a skin injury, a bone disorder and degenerative arthritis, etc.

The present invention also provides a pharmaceutical composition for preventing or treating an immune disorder comprising the composition of the present invention and mesenchymal stem cells as active ingredients.

The term "immune disorder" as used herein refers to any pathological state in which a tissue is damaged due to an excessive or undesired immune response. Accordingly, the term "immunological disorder" has the same meaning as "hyperactive immune disorder", and "composition for preventing or treating an immune disorder" has the same meaning as "immunosuppressive agent".

Such immune disorders include, but are not limited to, graft-versus-host disease, graft rejection, a chronic inflammatory disorder, inflammatory pain, neuropathic pain, a chronic obstructive pulmonary disorder (COPD) and an autoimmune disorder.

The term "autoimmune disorder" as used herein refers to a pathological state that occurs when immune cells attack the self without distinguishing the self from foreign substances. The autoimmune disorders include, but not limited to, rheumatoid arthritis, Hashimoto's thyroiditis, Grave's disease, multiple sclerosis, scleroderma, myasthenia gravis, type I diabetes, allergic encephalomyelitis, glomerulonephritis, vitiligo, Bechet's disease, Crohn's disease, ankylosing spondylitis, thrombocytopenic purpura, pemphigus vulgaris, autoimmune anemia, adrenoleukodystrophy (ALD), and systemic lupus erythematosus (SLE).

A pharmaceutical composition according to the present invention may further comprise a pharmaceutically acceptable additive in addition to the above-mentioned active ingredient.

In addition, the pharmaceutical composition may be formulated into a unit dosage form suitable for administration to a patient's body by a conventional method in the field of pharmacy. Formulations suitable for such purpose include solutions or suspensions as parenterally administered preparations, or ointments as topical preparations. When the composition of the present invention is formulated, fillers, extenders, binders, humectants, disintegrants, diluents such as surfactants, excipients or the like may be used together.

The dose of the composition may be about 1 μg to 50 mg per 1 kg body weight with respect to the total composition, and the dose for adults of the therapeutic cells such as immune cells and stem cells may be 1 to $10^8$, 10 to $10^5$, and $10^2$ to $10^3$ per day. The above administration can be conducted once or in divided doses several times a day. However, the doses and the number of administrations may be determined in consideration of the factors such as the patient's body weight, age and sex as well as the severity of a disease and the administration route.

**Mode for the Invention**

Hereinafter, the present invention will be described in detail with reference to the following Examples, Comparative Examples and Experimental Examples. However, the following Examples, Comparative Examples and Experimental Examples are intended to illustrate the present invention, and the scope of the present invention is not limited thereto.

**Examples 1 to 3. Preparation of medium compositions for cryopreservation**

To prepare a medium composition for cryopreservation, 5 ml of DMSO, 25 ml of dextran 40, 50 ml of Albumin Injection, and 20 ml of RPMI1640 as a cell culture medium were mixed (Example 1). In Examples 2 and 3, medium compositions for cryopreservation containing dimethyl sulfoxide (DMSO, BIONICHE PHARMA, USA), dextran 40 (Dai Han Pharm. Co., Ltd.), Albumin Injection (Green Cross, Korea) and RPMI1640 (Gibco, USA) were also prepared by the same method as in Example 1 but in the amounts shown in Table 1 below.

**Comparative Examples 1 to 6. Preparation of medium compositions for cryopreservation**

As the Comparative Examples to the above Examples, 5 ml of DMSO and 95 ml of Albumin Injection were mixed to prepare a medium composition for cryopreservation (Comparative Example 1). In Comparative Examples 2 to 6, medium compositions for cryopreservation containing dimethyl sulfoxide (DMSO, BIONICHE PHARMA, USA), dextran 40 (Dai Han Pharm. Co., Ltd.), Albumin Injection (Green Cross, Korea) and RPMI1640 (Gibco, USA) were also prepared by the same method as in Example 1 but in the amounts shown in Table 1 below.

[Table 1]

| | Example 1 | Example 2 | Example 3 | Comparative Example 1 | Comparative Example 2 | Comparative Example 3 | Comparative Example 4 | Comparative Example 5 | Comparative Example 6 |
|---|---|---|---|---|---|---|---|---|---|
| DMSO (v/v%) | 5 | 5 | 5 | 5 | 5 | 5 | 5 | 5 | 5 |
| Dextran 40 (v/v%) | 25 | 25 | 25 | - | 25 | 25 | 25 | 25 | 25 |
| Albumin Injection (v/v%) | 50 | 35 | 20 | 95 | 70 | 10 | 5 | 1 | 0 |
| RPMI1640 (v/v%) | 20 | 35 | 50 | - | - | 60 | 65 | 69 | 70 |

**Experimental Example 1. Evaluation of NK cells after freezing and thawing**

**1.1. Freezing and thawing of NK cells**

NK cells were frozen in the medium compositions for cryopreservation prepared in the Examples 1 to 3 and Comparative Examples 1 to 6 and then thawed to evaluate the medium compositions for cryopreservation.

First, mononuclear cells were isolated from human peripheral blood (Seoul National University Hospital, Korea). CD3-positive cells were removed from the isolated mononuclear cells using the CliniMACS system, and the resultants were used as seed cells. Meanwhile, the mononuclear cells irradiated with gamma rays at 2,000 cGy were used as supporting cells for NK cell culture. The seed cells and supporting cells were cultured in CellGro® SCGM medium (CellGenix, Germany) supplemented with 500 IU/ml of IL-2, 10 $\mu$g/ml of anti-CD3 antibody OKT3 and 1 v/v% of serum with the concentration maintainted at $0.5 \times 10^6$ to $1 \times 10^6$ cells/ml. The culture was carried out at under the condition of 37°C and 5% $CO_2$ for 14 to 21 days.

The cultured NK cells were collected and centrifuged under the condition of 4°C and 1,500 rpm for 10 minutes. The obtained pellet was washed with RPMI 1640 medium and the number of cells was counted. The cells were dispensed into 15 ml tubes at $1 \times 10^8$ cells/tube, and then centrifuged under the same condition as above to obtain pellets. The obtained pellets were frozen in the compositions for cryopreservation prepared in Examples 1 to 3 and Comparative Examples 1 to 6.

The cells were subjected to controlled rate freezing (CRF) under the condition shown in Table 2 below, and the frozen cells were stored in a vapor LN2 tank for a certain period of time to perform cell freezing. The frozen cells were collected and thawed rapidly in a constant temperature bath at 37°C. The cells were suspended in RPMI1640 medium supplemented with 10% fetal bovine serum, corresponding to 10 times the cell volume, which were then centrifuged under the condition of 4°C and 1,200 rpm for 10 minutes to obtain cells.

[Table 2]

| 0 step | End Temp. +4°C |
|---|---|
| 1 step | End Temp. -8°C; Slope -1°C/minute |
| 2 step | End Temp. -65°C; Slope -25°C/minute |
| 3 step | End Temp. -14°C; Slope +15°C/minute |
| 4 step | End Temp. -45°C; Slope -1°C/minute |
| 5 step | End Temp. -90°C; Slope -10.00°C/minute |

**1.2. Culture of NK cells**

The obtained cells were resuspended in RPMI1640 medium supplemented with 500 IU/ml of IL-2 and 10% fetal bovine serum to obtain a concentration of $2 \times 10^6$ cells/ml. The resuspended cells (10 ml) were dispensed in a T75 flask, and on the third day of culture, an equal volume of medium was added and cultured for 7 days.

**1.3. Examination of viable cell numbers and viability rates**

The numbers of viable cells and viability rates of the cultured NK cells were measured using ADAM cell counter Accustain kit (DigitalBio) according to the manufacturer's manual.

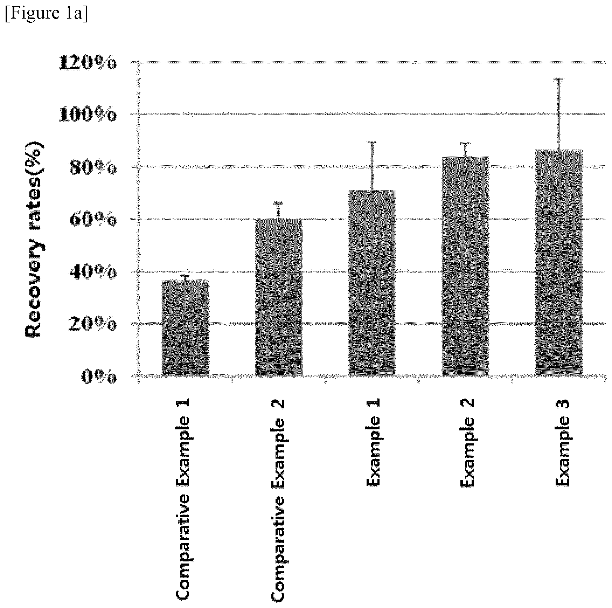

First, the cells cultured in Experimental Example 1.2 were counted and diluted with PBS to a concentration of $1 \times 10^6$ cells/ml. The diluted cells were dispensed into two wells of a round bottomed 96-well plate at 14 $\mu$l/well. 14 $\mu$l of T solution by which total number of cells can be measured was added to one of the two wells and mixed with diluted cells, and then 14 $\mu$l of the mixture was collected and put into the T panel of a counter chip. Meanwhile, 14 $\mu$l of N solution by which the number of dead cells can be measured was added to the other of the two wells and mixed with the diluted cells, and then 14 $\mu$l of the mixture was collected and put into the N panel of the counter chip. The chip was inserted into an ADAM counter and the number of viable cells was measured. The results are shown in Figs. 1a to 1d.

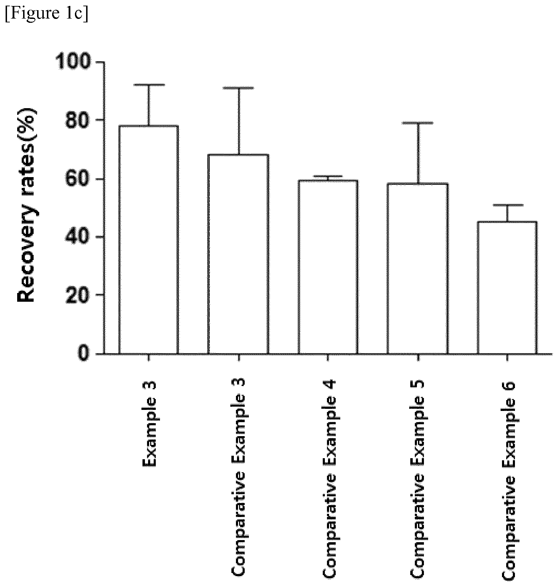

The recovery rates after thawing of the frozen cells and then culturing with IL-2 shown in Figs. 1a and 1c were shown as the percentages of the numbers of viable cells after thawing and culturing with IL-2 based on the numbers of cells in the vial at the time of freezing, which were shown in Tables 3 and 4 below.

[Table 3]

| | Average | Standard deviation |
|---|---|---|
| Comparative Example 1 | 36 | 2 |
| Comparative Example 2 | 60 | 5.8 |
| Example 1 | 72 | 18.1 |
| Example 2 | 82 | 6.2 |
| Example 3 | 85 | 27.6 |

[Table 4]

| | Average | Standard deviation |
|---|---|---|
| Example 3 | 78.5 | 13.6 |
| Comparative Example 3 | 67.6 | 23.1 |
| Comparative Example 4 | 58.9 | 2.4 |
| Comparative Example 5 | 57.8 | 20.8 |
| Comparative Example 6 | 45.0 | 6.5 |

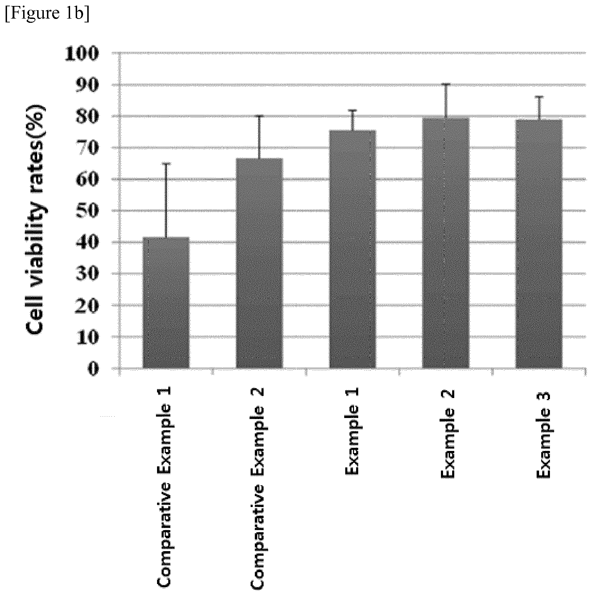

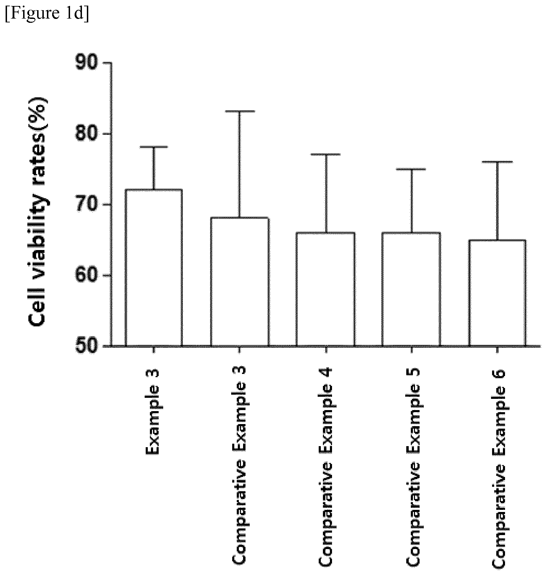

Meanwhile, the recovery rates after thawing of the frozen cells and then culturing with IL-2 shown in Figs. 1b and 1d were shown as the percentages of the numbers of viable cells based on the total numbers of cells, which were shown in Tables 5 and 6 below.

[Table 5]

| | Average | Standard deviation |
|---|---|---|
| Comparative Example 1 | 42 | 23 |
| Comparative Example 2 | 67 | 13 |
| Example 1 | 76 | 6 |
| Example 2 | 80 | 11 |
| Example 3 | 79 | 7 |

[Table 6]

| | Average | Standard deviation |
|---|---|---|
| Example 3 | 72 | 6 |
| Comparative Example 3 | 68 | 15 |
| Comparative Example 4 | 66 | 11 |
| Comparative Example 5 | 66 | 9 |
| Comparative Example 6 | 65 | 11 |

As shown in Figs. 1a to 1d and Tables 3 to 6, when the medium compositions for cryopreservation of Examples 1 to 3 were used after 7 days of culturing, the cell recovery rates and viability rates were as high as 70% or more. Meanwhile, the medium compositions for cryopreservation containing DMSO and Albumin Injection (Comparative Example 1), DMSO, dextran 40, and Albumin Injection (Comparative Example 2), DMSO, dextran 40, and Albumin Injection of less than 15 v/v% and RPMI1640 (Comparative Examples 3 to 6) resulted in low cell recovery rates and viability rates.

**1.4. Evaluation of NK cell activity**

In order to examine the cellular activities of NK cells frozen and thawed in the frozen media of Examples 1 to 3 and Comparative Examples 1 to 6, tumor cells were treated with NK cells and cytocidal ability on tumor cells was examined. Herein, the activities of the NK cells obtained immediately after thawing in Experimental Example 1.1 and those of the NK cells obtained after culturing in Experimental Example 1.2 were evaluated, respectively.

First, K562 cells (ATCC, USA), which are tumor cells, were cultured in RPMI1640 medium supplemented with 10% fetal bovine serum under the condition of 37°C and 5% $CO_2$. The cultured cells were recovered by trypsinization and suspended in the culture medium to a concentration of 1 x $10^6$ cells/ml. Then 1 mM calcein AM (Life Technologies, USA) was added to obtain a final concentration of 30 $\mu$M. The cells were cultured under the condition of 37°C and 5% $CO_2$ for 1 hour and the tumor cells were labeled with fluorescence.

The labeled tumor cells were washed with 10 ml of the culture medium, resuspended at a concentration of 1 x $10^5$ cells/ml, and then dispensed into a 96-well round bottomed plate at 100 $\mu$l/well. The cells obtained immediately after thawing and after culturing in Experimental Example 1.1 and Experimental Example 1.2 were mixed such that the ratios of the NK cells (effector cell, E) and K562 cells (target cell, T) were 10: 1, 3: 1 and 0.3: 1 each, which then added to the plate at 100 $\mu$l/well. Herein, for spontaneous release, the tumor cells were added with an equal amount of medium, and for maximal release, the tumor cells were added with an equal amount of 2% Triton X-100.

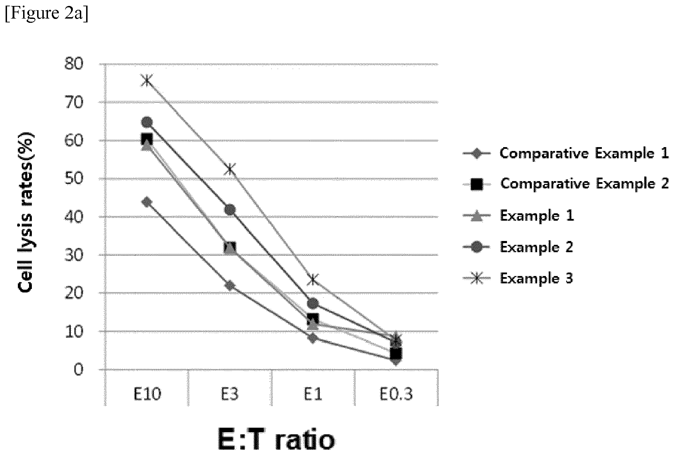

The mixtures were reacted under the condition of 37°C and 5% $CO_2$ for 4 hours, and then centrifuged at 4°C at 2,000 rpm for 3 minutes. Then, 100 $\mu$l of the obtained supernatant was transferred to a black well plate and measured with a fluorescence detector under the condition of 385 nm/450 nm. Using the fluorescence measurement values thus obtained, the dissolution rates (%) of tumor cells by NK cells were calculated by the following Equation (1) and shown in Figs. 2a to 2d, which were shown in Tables 7 to 10 below. The number of the natural killer cell donor is 6 in Table 7, and that of the natural killer cell donor is 3 in Table 8.

[Equation 1]

$$\text{Dissolution rate (\%)} = \frac{\text{Sample well average fluorescence value} - \text{Spontaneous release well average fluorescence value}}{\text{Maximum release well average fluorescence value} - \text{Spontaneous release well average fluorescence value}} \times 100$$

[Table 7]

| Immediately after thawing | Comparative Example 1 | Comparative Example 2 | Example 1 | Example 2 | Example 3 |
|---|---|---|---|---|---|
| E10 | 44 | 60 | 59 | 65 | 76 |
| E3 | 22 | 32 | 32 | 42 | 52 |
| E1 | 8 | 13 | 12 | 17 | 24 |
| E0.3 | 2 | 4 | 9 | 7 | 8 |

[Table 8]

| Immediately after thawing | Example 3 | Comparative Example 3 | Comparative Example 4 | Comparative Example 5 | Comparative Example 6 |
|---|---|---|---|---|---|
| E30 | 90 | 84 | 82 | 77 | 66 |
| E10 | 65 | 53 | 50 | 47 | 37 |
| E3 | 33 | 24 | 21 | 21 | 16 |
| E1 | 13 | 10 | 7 | 9 | 6 |
| E0.3 | 5 | 5 | 2 | 3 | 4 |

[Table 9]

| After culture | Comparative Example 1 | Comparative Example 2 | Example 1 | Example 2 | Example 3 |
|---|---|---|---|---|---|
| E10 | 84 | 87 | 86 | 86 | 88 |
| E3 | 68 | 82 | 77 | 76 | 77 |
| E1 | 45 | 61 | 52 | 46 | 45 |
| E0.3 | 17 | 28 | 21 | 19 | 18 |

[Table 10]

| After culture | Example 3 | Comparative Example 3 | Comparative Example 4 | Comparative Example 5 | Comparative Example 6 |
|---|---|---|---|---|---|
| E30 | 84 | 81 | 84 | 82 | 80 |
| E10 | 83 | 82 | 82 | 77 | 73 |
| E3 | 76 | 71 | 67 | 65 | 56 |
| E1 | 50 | 41 | 35 | 34 | 29 |
| E0.3 | 22 | 14 | 13 | 14 | 10 |

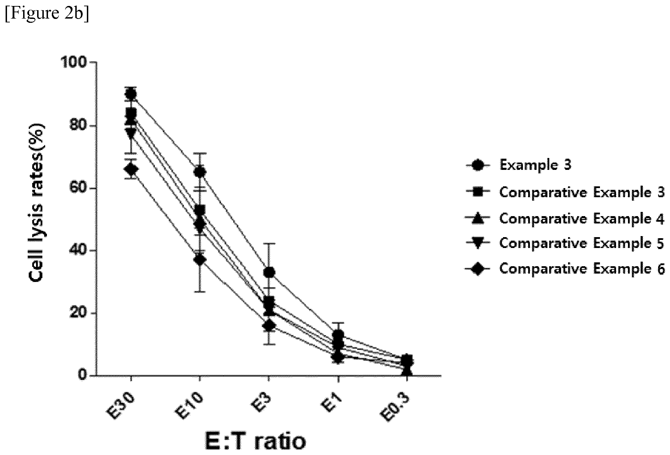

As shown in Figs. 2a and 2b and Tables 7 and 8, it was found that the cytotoxicity against tumors immediately after thawing of the frozen cells was lower when the albumin content was either 10% or less or 70% or more as compared to Examples. In general, it is known that when activated immune cells are frozen, the functions of the cells are reduced due to the decrease in the activities of the cells. However, it was found that the thawed cells maintained their activities of various degrees depending on the composition of the cryopreservation medium. In particular, it was found that in Example 3, the highest NK cell activity was maintained when the cells were thawed after freezing.

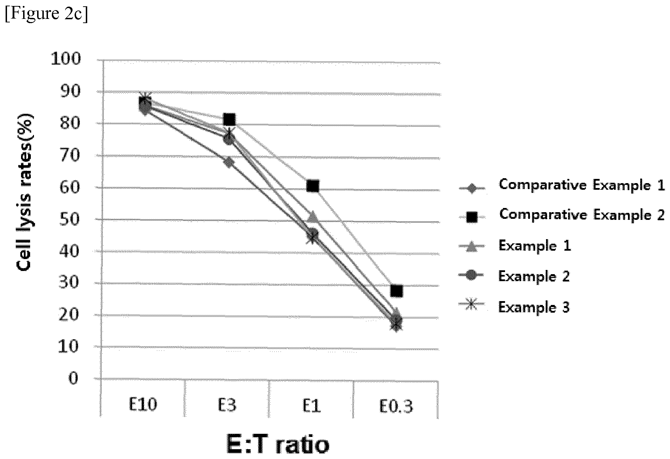

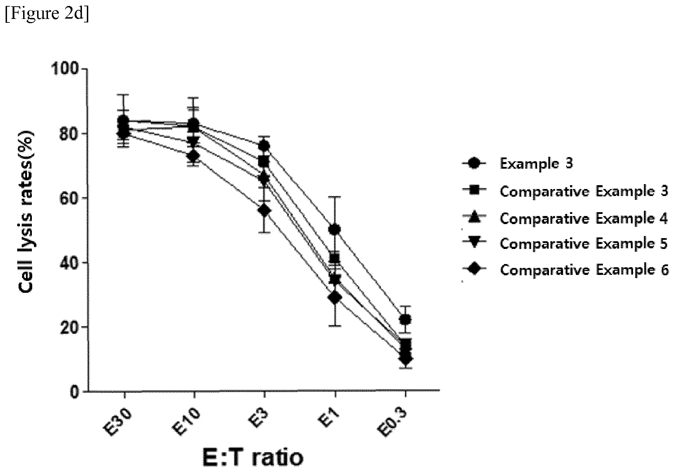

Meanwhile, the results of examining the cytotoxicity of the frozen cells after culturing for a certain period of time against the tumor cells were shown in Figs. 2c and 2d and Tables 9 and 10 above, which indicated that all Examples and Comparative Examples showed similar cytotoxicity. It was contemplated that the results were owing to the recovery of the activity of NK cells during the culture which was reduced by IL-2 added to the medium.

**Experimental Example 2. Evaluation of tumor cells after thawing**

It was examined whether the medium compositions for cryopreservation prepared in Examples 1 to 3 and Comparative Examples 1 to 6 could be used for freezing tumor cells as well.

First, human T lymphoma cells HuT78 (ATCC, USA) and human leukemia cells K562 (ATCC, USA) were respectively cultured in RPMI1640 medium supplemented with 10% fetal bovine serum and RPMI1640 medium supplemented 20% fetal bovine serum under the condition of 37°C and 5% $CO_2$. The cultured cells were recovered and suspended in each culture medium. Then, the HuT78 cells and the K562 cells were placed in vials and frozen at the concentrations of $1 \times 10^7$ cells/ml and $4 \times 10^6$ cells/ml, respectively. The cell freezing was performed by the same method as described in Experimental Example 1.1 above. The frozen cells were resuspended in each culture medium, and the cells were subcultured to obtain the concentration of $1 \times 10^6$ cells/ml and cultured under the condition of 37°C and 5% $CO_2$ for 4 days.

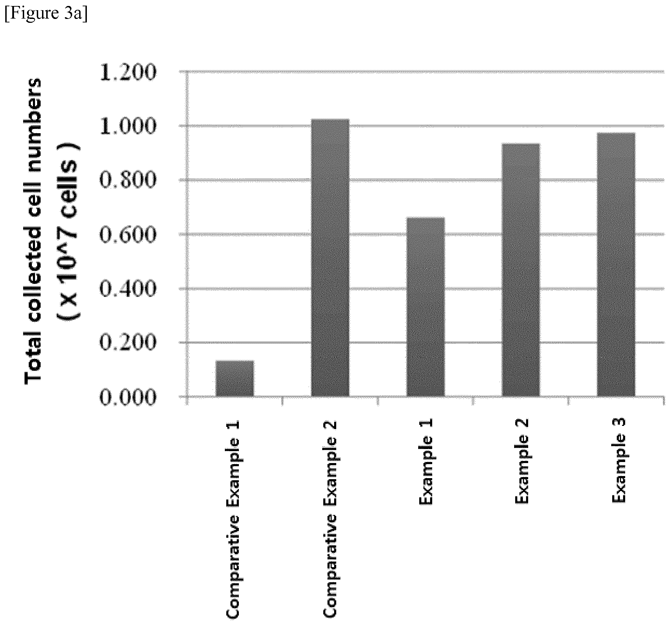

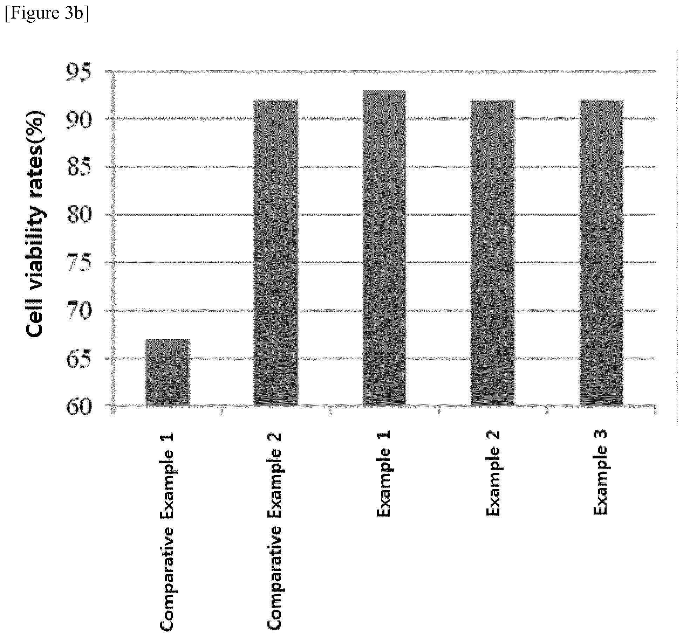

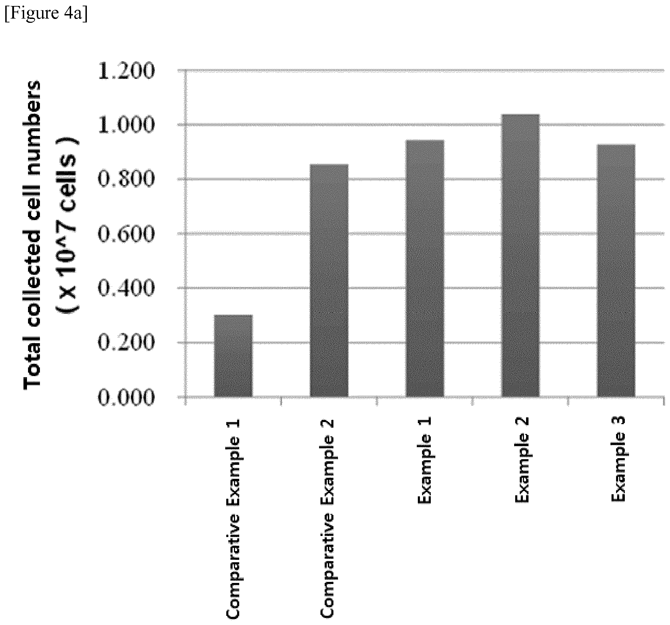

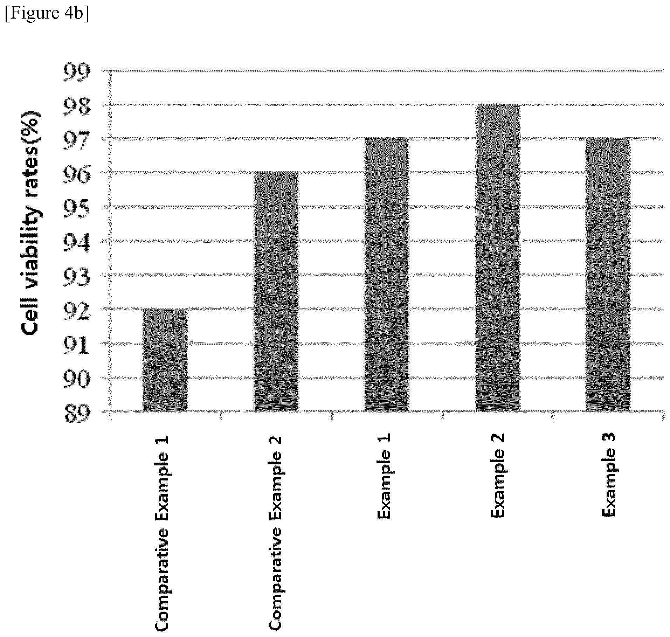

The cultured cells were measured for the viable cell numbers and viability rates by the same method as in Experimental Example 1.3. The viable cell numbers and viability rates of HuT78 cells were shown in Figs. 3a and 3b, and those of K562 cells were shown in Figs. 4a and 4b.

As a result, it was found that the viable cell numbers and viability rates of tumor cells HuT78 and K562 cells in the medium compositions for cryopreservation of Examples 1 to 3 and in the medium composition for cryopreservation of Comparative Example 2 were similar as shown in Figs. 3a to 4b. Meanwhile, the medium composition for cryopreservation of Comparative Example 1 showed a remarkably low viable cell number and viability rate.

**Claims**

1.  A medium composition for cryopreservation of cells comprising 15 to 55 v/v% of albumin, 20 to 30 v/v% of dextran, 2 to 8 v/v% of dimethyl sulfoxide and 15 to 55 v/v% of a cell culture medium based on the total volume of the composition.

2.  The medium composition of claim 1, wherein the albumin is comprised in an amount of 18 to 40 v/v%.

3.  The medium composition of claim 1, wherein the dextran is comprised in an amount of 23 to 27 v/v%.

4.  The medium composition of claim 1, wherein the dimethyl sulfoxide is comprised in an amount of 4 to 6 v/v%.

5.  The medium composition of claim 1, wherein the cell culture medium is comprised in an amount of 30 to 52 v/v%.

6.  The medium composition of claim 1, wherein the cells are animal cells.

7.  The medium composition of claim 6, wherein the animal cells are immune cells.

8.  The medium composition of claim 7, wherein the immune cells are natural killer cells.

9.  The medium composition of claim 1, wherein the composition is administered directly to a subject with the cells.

10. A method for cryopreservation of cells, comprising the step of freezing cells to which the medium composition of claim 1 is added.

11. A pharmaceutical composition for preventing or treating a cancer or infectious disorder comprising the medium composition of claim 1 and immune cells as active ingredients.

12. The pharmaceutical composition of claim 11, wherein the immune cells are natural killer cells.

13. A pharmaceutical composition for preventing or treating a degenerative disorder comprising the medium composition of claim 1 and stem cells as active ingredients.

14. The composition of claim 13, wherein the stem cells are mesenchymal stem cells.

15. The composition of claim 13, wherein the degenerative disorder is any one selected from the group consisting of a cranial nerve disorder, an ischemic disorder, a skin injury, a bone disorder and degenerative arthritis.

16. A pharmaceutical composition for preventing or treating an immune disorder comprising the medium composition of claim 1 and mesenchymal stem cells as active ingredients, wherein the immune disorder is selected from the group consisting of a graft-versus-host disorder, graft rejection, an autoimmune disorder, a chronic inflammatory disorder, inflammatory pain, neuropathic pain and a chronic obstructive pulmonary disorder (COPD).

[Figure 1a]

[Figure 1b]

[Figure 1c]

[Figure 1d]

[Figure 2a]

[Figure 2b]

[Figure 2c]

[Figure 2d]

[Figure 3a]

[Figure 3b]

[Figure 4a]

[Figure 4b]

## INTERNATIONAL SEARCH REPORT

| International application No. |
|---|
| **PCT/KR2017/000859** |

**A. CLASSIFICATION OF SUBJECT MATTER**

*A01N 1/02(2006.01)i, C12N 5/0783(2010.01)i, C12N 5/0775(2010.01)i, A61K 35/17(2014.01)i, A61K 35/28(2006.01)i*

According to International Patent Classification (IPC) or to both national classification and IPC

**B. FIELDS SEARCHED**

Minimum documentation searched (classification system followed by classification symbols)
A01N 1/02; A61K 35/56; C12N 5/0789; C12N 5/0783; C12N 5/0775; A61K 35/17; A61K 35/28

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: cryopreservation, medium, albumin, dextran, dimethyl sulfoxide, nonserum

**C. DOCUMENTS CONSIDERED TO BE RELEVANT**

| Category* | Citation of document, with indication, where appropriate, of the relevant passages | Relevant to claim No. |
|---|---|---|
| X | US 2014-0220551 A1 (PRIMEGEN BIOTECH. LLC.) 07 August 2014 | 1-10 |
| Y | See paragraph [0004]; claims 1-8. | 11-16 |
| Y | KR 10-2014-0123503 A (MOGAM INSTITUTE FOR BIOMEDICAL RESEARCH et al.) 22 October 2014 See abstract; claims 1 and 12-18. | 11,12 |
| Y | FARINI et al., "Clinical Applications of Mesenchymal Stem Cells in Chronic Diseases", Stem Cells International, vol. 2014, document ID 306573, pages 1-11 (2014) See abstract. | 13-16 |
| A | KR 10-2007-0103431 A (REGENETECH, INC.) 23 October 2007 See claims 1-3 and 9. | 1-16 |
| A | US 2015-0320031 A1 (PHARMACOSMOS A/S.) 12 November 2015 See claims 1, 16, 17, 28, 30 and 35. | 1-16 |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 28 APRIL 2017 (28.04.2017) | **01 MAY 2017 (01.05.2017)** |

| Name and mailing address of the ISA/KR | Authorized officer |
|---|---|
| Korean Intellectual Property Office Government Complex-Daejeon, 189 Seonsa-ro, Daejeon 302-701, Republic of Korea | |
| Facsimile No. +82-42-481-8578 | Telephone No. |

Form PCT/ISA/210 (second sheet) (January 2015)

**INTERNATIONAL SEARCH REPORT**
Information on patent family members

International application No.

**PCT/KR2017/000859**

| Patent document cited in search report | Publication date | Patent family member | Publication date |
|---|---|---|---|
| US 2014-0220551 A1 | 07/08/2014 | NONE | |
| KR 10-2014-0123503 A | 22/10/2014 | AU 2012-354438 A1 | 27/06/2013 |
| | | AU 2012-354438 B2 | 31/03/2016 |
| | | CN 104204194 A | 10/12/2014 |
| | | EP 2794859 A1 | 29/10/2014 |
| | | JP 2015-502756 A | 29/01/2015 |
| | | JP 6010136 B2 | 19/10/2016 |
| | | KR 10-1644984 B1 | 12/08/2016 |
| | | US 2015-0118207 A1 | 30/04/2015 |
| | | WO 2013-094988 A1 | 27/06/2013 |
| KR 10-2007-0103431 A | 23/10/2007 | CA 2596083 A1 | 03/08/2006 |
| | | CN 101142310 A | 12/03/2008 |
| | | EP 1859024 A2 | 28/11/2007 |
| | | IL 184700 A | 03/12/2007 |
| | | JP 2008-528034 A | 31/07/2008 |
| | | WO 2006-081435 A2 | 03/08/2006 |
| | | WO 2006-081435 A3 | 08/03/2007 |
| US 2015-0320031 A1 | 12/11/2015 | AR 093662 A1 | 17/06/2015 |
| | | AU 2013-351058 A1 | 28/05/2015 |
| | | CA 2891198 A1 | 05/06/2014 |
| | | CN 104918486 A | 16/09/2015 |
| | | EA 201591052 A1 | 30/09/2015 |
| | | EP 2934109 A1 | 28/10/2015 |
| | | IL 239027 A | 30/07/2015 |
| | | JP 2016-501873 A | 21/01/2016 |
| | | KR 10-2015-0095723 A | 21/08/2015 |
| | | MX 2015006617 A | 16/12/2015 |
| | | SG 11201504116 A | 29/06/2015 |
| | | WO 2014-083169 A1 | 05/06/2014 |

Form PCT/ISA/210 (patent family annex) (January 2015)